# EUROPEAN PATENT SPECIFICATION

(11) **EP 4 252 690 B1**
(45) Date of publication and mention of the grant of the patent: **28.08.2024**
(21) Application number: 23163049.2
(22) Date of filing: 21.03.2023
(51) Int. Cl.: A61B 18/12, A61B 18/14

(54) **ELECTROSURGICAL GENERATOR WITH DETECTION OF INSTRUMENT PLUG-IN**
ELEKTROCHIRURGISCHER GENERATOR MIT DETEKTION EINES INSTRUMENTENSTECKPLATZES
GÉNÉRATEUR ÉLECTROCHIRURGICAL AVEC DÉTECTION DE L'ENFICHAGE D'INSTRUMENT

(30) Priority: 31.03.2022 US 202263325945 P
(43) Date of publication of application: 04.10.2023
(73) Proprietor: Olympus Winter & Ibe GmbH, 22045 Hamburg (DE)
(72) Inventor: Kühne, Wolfgang, 16552 Schildow (DE); Dijkstra, Jelle, 12205 Berlin (DE)
(74) Representative: Glawe, Delfs, Moll

(56) References cited:
- WO-A1-97/24073
- US-A1- 2013 231 656
- US-A1- 2014 066 927
- US-A1- 2014 171 935

## Description

The invention concerns an electrosurgical generator. The electrosurgical generator is designed to output a high-frequency alternating voltage to an electrosurgical instrument. It comprises a control unit and an inverter for high-voltage that generates the high-frequency alternating voltage which is fed via an output connection to an output socket for connection of the electrosurgical instrument.

Electrosurgical generators find widespread use in surgical theatres. They are used for a variety of tasks in different fields of surgery, and various kinds and types of instruments are connected to the electrosurgical generator, said instruments being designed and configured specially for the surgical task to be accomplished. For proper and safe operation of the electrosurgical generator it is important that the generator detects when the electrosurgical instrument is plugged in. Generally there are two types known for such detection. The first kind is detecting whether an instrument is plugged in at all. The second kind is furthermore capable to recognize the type of instrument connected. The latter is quite important with respect to sophisticated instruments which have a memory on their own, the memory storing coded information which electrosurgical modes may be used with that particular instrument, and an even more sophisticated instrument may be even provided with an own microcontroller for additional functionality having an interface for communication with the electrosurgical generator.

Those detection features improve safety and user experience for the surgeons using the electrosurgical generator. In order to detect plugging of instruments several concepts are known.

A first rather simple concept is to split the socket into two parts which are connected with each other as soon as the instrument with its cable and a conducting pin at the end of the cable is plugged in. A second concept is to provide a pushbutton adjacent to the socket which is directly or indirectly pressed whenever the cable of the instrument with its pin is plugged in. A third concept provides a light barrier that detects the presence of the pin thereby allowing contactless detection. The fourth concept provides a distance sensor mounted at the rear side of the socket, thereby detecting when the instrument cable with its pin is plugged in. These concepts generally work, however they are complicated due to rather stringent isolation requirements that must be met in consideration of the high-voltage used for the instruments. Further, most of these concepts require at least one additional contact which is prone to wear and further needs proper isolation.

It is thus an object of the invention to provide an electrosurgical generator having an improved plugging-in detection of instruments.

The solution according to the invention is found in the features of the independent claim. Advantageous developments are the subject matter of the dependent claims.

In an electrosurgical generator designed to output a high-frequency alternating voltage to an electrosurgical instrument, comprising a control unit and an inverter for high voltage that generates a high-frequency alternating voltage which is fed via an output connection to an output socket for connection of the electrosurgical instrument, wherein a galvanic separator is provided isolating the output socket from the control unit, and wherein a detection unit is provided being configured to detect when the electrosurgical instrument is plugged in the output socket, it is provided according to the invention that the detection unit is a capacitive detector configured for detecting a capacitance of a plugged-in cable of the electrosurgical instrument.

The core of the invention is that by plugging in the instrument its cable inevitably forms a parasitic capacitance from the output socket to earth. Normally, a parasitic capacitance like the one of the instrument's cable is not desirable. The invention recognizes that this parasitic capacitance may be utilized in a useful manner. This is however complicated by the fact that the parasitic capacitance is usually small in value and varies from instrument to instrument. Further, the required galvanic isolation between the cable/socket and the interior circuits of the generator complicates a precise measurement of the parasitic capacitance. The value is typically in the range of about 5 to 30 pF only, making a precise measurement difficult. However, it was realized by the invention that a precise measurement of the capacitance is not required since in principle only a binary information suffices, namely whether an instrument cable is plugged-in or not. Without an instrument and its cable being connected to a socket, the capacitance of the corresponding pin of the socket will be very low. As soon as a cable is connected to the socket, the capacitance will be increased by the parasitic capacitance of the instrument and its cable. The value is, as already stated, rather low typically ranging about 5 - 30 pF. By determining, whether the total capacitance jumps by such amount, the invention achieves a qualitative detection when the instrument and its cable is being plugged in. Thereby, a rather simple and cost-effective plugging-in detection of the cable is realized. This can be further enhanced by connecting the capacitive detector to the output connection at an electrode-conductor of the output connection, preferably at the active electrode (AE). Such connection (exclusively) to the conductor for the electrode ("electrode-conductor") avoids any need for an additional wiring (sense wire) in the cable/socket, further contributing to simplicity and cost-effectiveness.

A few terms used should first be explained below:
In the field of electrosurgical generators, "high frequency" refers to frequencies typically in the range between 100 kHz and 4000 kHz.

"High voltage" typically refers to voltages up to 10 kV, preferably up to 4000 V.

A galvanic separator provides galvanic isolation, preventing any direct current flow between the output socket and the interior circuits of the generator, typically termed "secondary circuit" by persons skilled in this art. However, alternating current may flow via a capacitance.

Preferably, the capacitive detector can be simplified such as to being configured for detecting just a binary state, a first (high) capacitance state indicating the instrument's cable being plugged-in and a second (low) capacitance state indicating the instrument's cable being not plugged-in. By such a simplified detector configuration a minimum of hardware is required for the capacitive detector.

Preferably, the capacitive detector comprises a capacitive pick-up arranged at the output socket and a measuring circuit being configured to check a measured capacitance against a reference value. The capacitive pick-up supplies a signal to be measured to the measuring circuit. Thereby, the measuring circuit can be placed away from the capacitive pickup at the socket if desired. The reference value acts as a threshold value against which the measured capacitance can be checked, and accordingly the binary information that the instrument with its cable is plugged-in (measured total capacitance being above the reference value) or is not plugged-in (measured total capacitance being below said reference value). This is a rather simple and cost-effective way for determining the status of the instrument being plugged in or not.

In a preferred embodiment the measuring circuit is galvanically isolated from the capacitive pick-up. Thereby, the measuring circuit can be placed in the internal circuitry of the electrosurgical generator while maintaining proper isolation. To this end, the measuring circuit is preferably connected to the capacitive pick-up by a protective capacitor connected in series. Said protective series capacitor is separate from the isolation capacitor acting as galvanic isolator required by regulation to be present between the high-voltage generating inverter and the output socket. By providing such a separate protective series capacitor the measuring circuit is well protected and can be placed in the so-called "secondary circuit" (which relates to the control unit and other internal circuitry) of the electrosurgical generator. The capacitance of the pin of the socket (and the instrument with its cable if connected) is then connected in series with the protective capacitor, thereby limiting the total capacitance to that of said protective capacitor. This decreases the sensitivity of the circuit for the sake of proper protection. This is perfectly acceptable since according to the invention only a binary information is required, namely whether the instrument with its cable is connected or not.

Preferably, the protective capacitor is configured as a printed circuit board (PCB) structure. Using such a PCB structure is a space enclosed efficient way in order to create the series protective capacitor. Preferably, said PCB structure comprises two plates being opposite from each other and being isolated from each other, said plates may be formed by respective layers of the PCB. By virtue of this, no additional components are required other than the PCB and its conductive areas/layers acting as plates. This is sufficient in order to create the required capacitance which is typically about a few picofarads.

In an alternative preferred embodiment, the measuring circuit may be connected to the output socket, in particular directly i.e. without galvanic isolation or in other words galvanically, and an output signal emitted by the measuring circuit is fed to the control unit via a protected connection (which provides a galvanic separation). Thereby galvanic isolation between output socket and measurement circuit becomes superfluous (although it may be present anyway). Preferably, however is a direct connection of the pin of the output socket to the measurement circuit, thereby increasing sensitivity. However, the output of the measuring circuit must be transferred in a safe manner to the control unit, and in order to accomplish this the protected connection is provided. The protective connection is a connection enabled to transfer a signal but preferably allowing the potential of the measuring circuit to be floating. Thereby a sensitive direct measurement at the pin of the socket is realized and a safe transfer of the output of the measuring circuit to the control unit is achieved, too. This embodiment is particularly useful if the output socket concerned is already provided with a microcontroller having a local ground and a protected communication interface to the control unit (or the "secondary circuit").

Advantageously, the measuring circuit is configured for direct capacitance measurement, preferably formed by an integrated circuit. Such a direct measurement allows for an effective measurement with little effort, thereby usually providing robust measurement results. Further, integrated circuits are available being configured for direct measurement in a single chip. These allow for a reliable measurement with little effort in terms of space and cost.

Alternatively, the measuring circuit is configured for indirect capacitance measurement. By using an indirect measurement signals or circuitry used for another purpose and is already present may be employed in order to provide the desired measurement of capacitance, thereby minimizing the need of additional circuitry.

In an alternative preferred way, a low voltage AC signal may be injected into the output connection, and the measuring circuit is configured for measuring said low voltage AC signal. Thereby the often difficult measurement of leakage current can be transformed into a measurement of a voltage signal which can be accomplished in an efficient manner without requiring extensive circuitry. Usage of an AC signal avoids adverse effects of DC drift and further allows an often desired decoupling of potentials by using capacitors.

Preferably, the measuring circuit comprises a capacitive voltage divider connected between the output connection and a local ground at the output socket, said capacitive voltage divider generating a low voltage signal to be measured. By virtue of such a configuration, inevitable parasitic capacitance between a local ground at the output socket and protective earth (PE) can be transformed into a useful tool for the required measurement.

Further preferably a, a resistive voltage divider is provided for providing a bias voltage for the measuring circuit. Such a resistive voltage divider provides in an efficient manner a bias voltage for the otherwise floating potential of the capacitive voltage divider. Such a fixed potential aids in subsequent signal conditioning, in particular by usage of an operational amplifier.

Advantageously, the measuring circuit comprises an operational amplifier being configured for setting different DC gain and AC gain, wherein the AC gain is at least ten times, preferably more than fifty times, higher than the DC gain which is preferably set to substantially unity. By selecting the AC gain much higher than the DC gain only the relevant frequencies for carrying out the measurement are amplified, thereby reducing noise floor and enhancing precision of measurement. This can be further improved by providing an optional blocking filter, preferably a notch filter, configured for blocking a switching frequency of a power supply of the electrosurgical generator. Thereby adverse effects of the power supply of the electrosurgical generator can be reduced with little effort. This enhances robustness of the measurement and therefore reliability of the inventive detection whether an instrument with its cable is plugged-in or not.

The invention is explained in more detail below with reference to an advantageous exemplary embodiment. In the figures:
- Fig. 1: shows an electrosurgical generator according to a first exemplary embodiment with an attached electrosurgical instrument;
- Fig. 2: shows a schematic functional diagram of the electrosurgical generator according to Fig. 1;
- Fig. 3: shows a part of a schematic functional diagram according to a second exemplary embodiment; and
- Fig. 4: shows a circuit diagram of an exemplary embodiment of a measurement circuit and its connection.

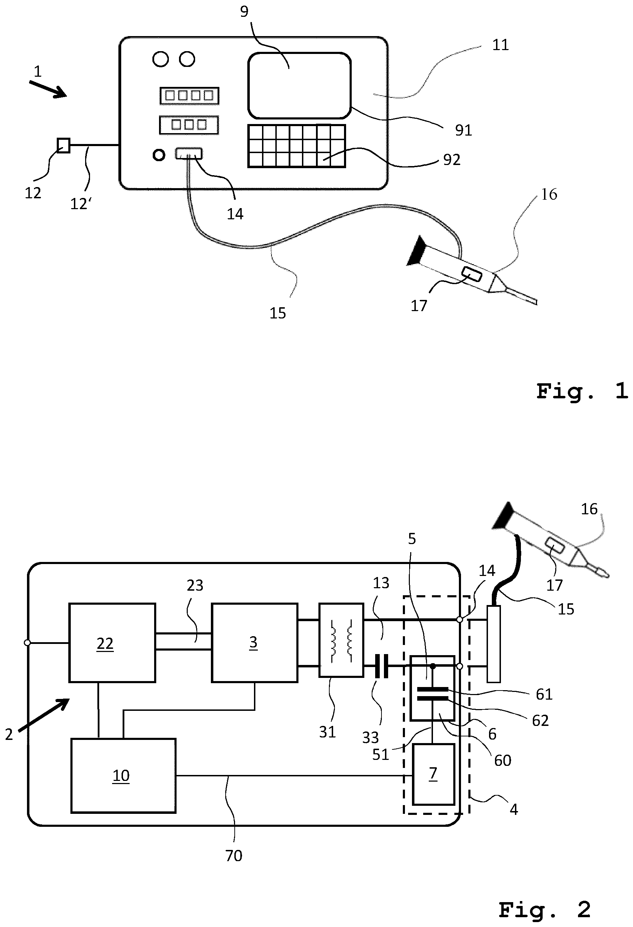

An electrosurgical generator according to a first exemplary embodiment of the invention is illustrated in Fig. 1. The electrosurgical generator, identified as a whole by reference numeral 1, comprises a housing 11 having an output socket 14 for connection of an electrosurgical instrument 16. A power supply cable 12' is provided which can be connected to an electrical power source 12 which may be an electricity grid, like the AC mains in a building, or an off-grid source of electric energy, like a 12 Volt or 24 Volt battery in a vehicle or mobile hospital. Further, a user interface 9 is provided comprising a display 91 and keyboard or touchscreen section 92 for inputs by the user. The display 91 shows information concerning the inputs made by the user and the status of the electrosurgical generator 1. By virtue of the user interface 9 the user can issue directions and commands to a control unit 10 which controls operation of the electrosurgical generator 1 and its components, including frequency and voltage of the AC voltage emitted by the output socket 14 as well as modes of operation.

Said electrosurgical instrument 16 comprises a cable 15 which is to be plugged-in into the output socket 14 in order to supply the high-frequency alternating voltage for operation of the electrosurgical instrument 16.

Fig. 2 shows a schematic functional diagram of an internal circuitry 2 of the electrosurgical generator 1. It comprises a power supply unit 22 that is fed by electrical energy from the power supply 12 by the supply cable 12', the power supply unit 22 feeding a DC bus 23 connected to an inverter 3 configured for generating high-frequency alternating current in a high-voltage range of a few kilovolts. Operation of the inverter 3 is governed by the control unit 10 which in turn is connected with the user interface 9 such that the user can issue directions and commands for operation of the electrosurgical generator 1, the control unit 10 generates corresponding control signals and governs the relevant components of the internal circuitry 2 according to these instructions and commands.

The high-frequency output emitted by the inverter 3 is applied to an isolation transformer 31 stepping up the voltage to a high-voltage, and the resulting high-voltage is routed via an output connection 13 to the output socket 14. The output connection 13 typically comprises two conductors, one for a neutral electrode NE and one other for an active electrode AE. At least the conductor of one of electrodes NE and AE, in this embodiment the active electrode AE, comprises a series capacitor 33 for galvanic isolation and blocking of any DC current to the output socket 14. The electrosurgical instrument 16 with its cable 15 can be plugged into the output socket 14.

There may be various electrosurgical instrument 16 of different kinds. In order to provide identification, the electrosurgical instrument 16 is optionally equipped with a memory 17 comprising data identifying the electrosurgical instrument 16 and allowing the electrosurgical generator 1 to determine which modes of operation could be used with respect to the electrosurgical instrument 16 actually plugged into the output socket 14.

Further, a capacitive detector 4 is provided at the output socket 14. In Fig. 2 a first embodiment of said capacitive detector 4 is depicted. Said first embodiment of the capacitive detector 4 comprises a pickup 5 feeding a signal to a measurement circuit 7. The pickup 5 comprises a connection 50 and a protective capacitor 6, a first end of the connection 50 being connected to the conductor of the active electrode AE and the other, second end being connected to a protective capacitance 6 connected in series. The protective capacitor 6 comprises a printed circuit board (PCB) 60 on which two plates 61 and 62 are formed. In this embodiment, the two plates 61 and 62 are at different layers of the PCB 60, i.e. a top layer and a middle (or bottom) layer. Each of the plates 61, 62 has preferably a rounded, in particular a circular shape, the two plates 61, 62 being placed in a stacked configuration and being spaced apart from each other by a predefined distance, in this embodiment determined by the thickness of the PCB 60 (or a fraction thereof if a middle layer is employed for at least one of the plates). Thereby the two plates 61 and 62 form a capacitance on the PCB 60 in a rather flat and space efficient manner. From the protective capacitor 6 a signal line 51 leads to an input of the measuring circuit 7. The measuring circuit 7 is decoupled from the output socket 14 by virtue of the protective capacitor 6 providing galvanic separation. Therefore, the measuring circuit 7 belongs to the internal circuitry 2 of the electrosurgical generator 1.

The measuring circuit 7 in this embodiment is configured for direct capacitance measurement, preferably by a dedicated integrated circuit as it is commercially available (e.g. MSP430FR2512IRHL of Texas Instruments, Inc.). If the measured capacitance is higher than the threshold value, then this acknowledges detection of the instrument 16 with its cable 15 being plugged-in to the socket 14, and a corresponding signal is emitted by a signal line 70 to the control unit 10.

This may be illustrated by the following example: typically the cable 15 of the electrosurgical instrument 16 has a capacitance of about 5.9 pF to earth. The capacitance of the output socket 14 with its pin is usually in the range between 1 to 10 pF. Further, the protective capacitor 6 being connected in series shall have a capacitance of a few picofarads, e.g. 10 pF. Due to a series configuration of the parasitic impedance of the cable 15 and the protective capacitor 6, the resulting total capacitance is limited to by the capacitance of the protective capacitor 6. This decreases the sensitivity of the circuit, however this is not a problem since an exact measurement of the capacitance is not required rather than just a binary information stating whether the surgical instrument 16 with its cable 15 is plugged-in into the output socket 14 or not.

With no instrument 16 plugged-in, the total measured capacitance is determined by e.g. 5 pF for the pin of the output socket 14 to protective earth (PE) in a series configuration with the protective capacitor 6 having a capacity of 10 pF resulting in a total capacitance of 3.33 pF. - Conversely, with the instrument 16 and its cable 15 and plugged into the output socket 14, the added capacitance of the pin of the output socket 14 to earth and cable 15 of the electrosurgical incident 16 to earth is 10.9 pF, again to be combined in a series configuration with the protective capacitor 6 having a capacity of 10 pF resulting in a total capacitance of 5.2 pF. This is a capacitance increase of nearly 60% which is easily detectable by comparing with a properly set threshold, e.g. set at 4 pF in the context of the present example. If the direct measured capacitance is higher than 4 pF then it means that the instrument 16 with its cable 15 is plugged-in to the socket 14, as it is below it does mean that it is not plugged-in.

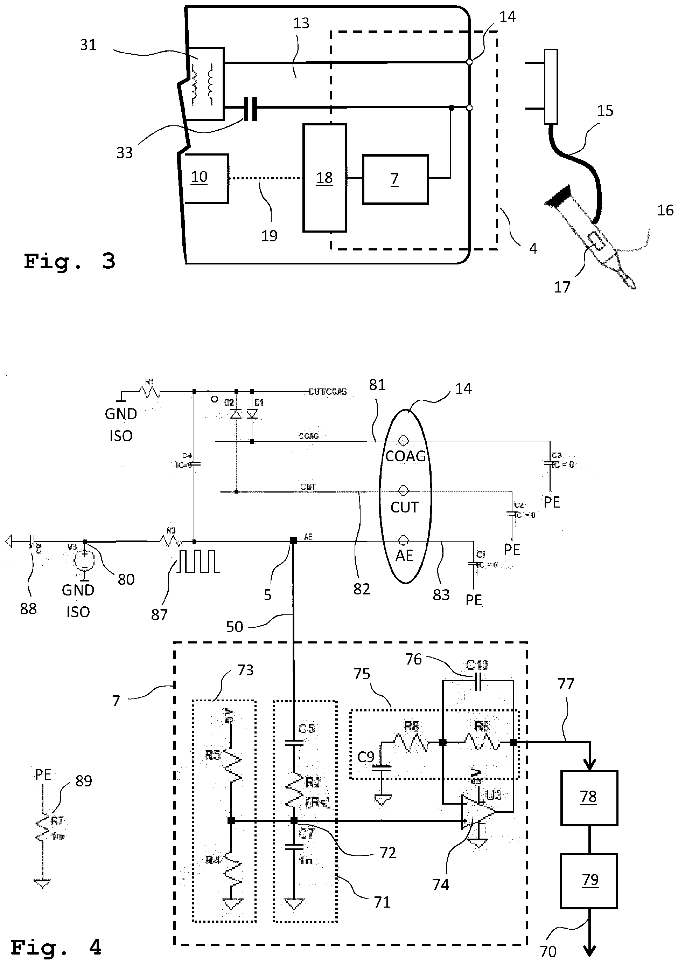

A second exemplary embodiment is shown partly in Fig. 3. The general constitution of the electrosurgical generator 1 is the same as that of the first exemplary embodiment, however the configuration of the capacitive detector for the measuring circuit 7 is different, and the relevant section of the electrosurgical generator 1 is depicted in Fig. 3. In this second exemplary embodiment, the measuring circuit 7 is not decoupled from the output socket 14 by protective capacitor 6 as it was the case in the first exemplary embodiment. Instead, the measuring circuit 7 is directly connected to the pin of the output socket 14. This, however, requires the measurement circuit to be isolated from the internal circuitry 2. So the information signal concerning the measured capacitance must be transferred to the control unit 10 by a safe communication interface. This may be a local microcontroller 18 for the output socket 14 that is connected to the control unit 10 via a special protected communication link 19 providing galvanic separation. If such devices are already present, then thereby the capacitance signal as measured by the measurement circuit 7 can be easily transferred to the control unit 10 in a safe manner. The direct connection of the measurement circuit 7 allows for convenient and more precise measurement of capacity. There is, however, a drawback in that the measurements circuit 7 is at a floating potential with respect to the internal circuitry 2.

A measurement circuit 7 configured to indirectly measure the capacitance is shown in Fig. 4. Instead of measuring directly the change in capacitance connected to the pin of the output socket 14, the measurement circuit 7 is provided to measure a +12/-12 Volt toggle voltage 87 that is generated for detecting handswitch presses. A variant of the output socket 14 having a three-prong connector to the electrosurgical instrument 16 is shown in Fig. 4. As the three parallel lines in the upper right-hand corner of that figure show, the output socket 14 comprises three lines, line 83 for the active electrode (AE), line 82 for determining a cut-mode (CUT) and line 81 for determining a coagulation mode for sealing vessels ("Coag"). In order to determine the status of a handswitch toggling between cut and coag mode, a +12/-12 Volt toggle voltage, symbolized by the square wave train 87, is provided by toggle voltage injector 80.

This toggle voltage is also present at the line 83 for the active electrode AE. This is where the pickup 5 of the capacitance detector 4 is connected at. The lower portion of Fig. 4 shows the measurement circuit 7 and its pickup 5. Accordingly, a line 50 of the pickup 5 is routed into the measurement circuit 7 directly and supplied to a capacitive voltage divider 71 comprising capacitors C5 and C7, wherein line 50 connects to capacitor C5 and capacitor C7 is connected to a local ground (symbolized in the figures by a triangle on its tip). Capacitors C5 and C7 are connected at a midpoint 72 which is supplied to a positive input of an operational amplifier 74. Further, a resistive voltage divider 73 is provided connected between supply voltage (typically 5 Volt) and local ground. Its midpoint is connected to midpoint 72 of the capacitive voltage divider 71 thereby providing a bias voltage for said midpoint 72 of the capacitive voltage divider 71 and for the positive input of said operational amplifier 74, the bias voltage being preferably set at preferably approximately 50% of the supply voltage (e.g. 2.5 Volt). A feedback circuit 75 is provided for the operational amplifier 74 and connected to its output and negative input. The feedback circuit 75 comprises resistors R6, R8 and C9 setting the gain of the operational amplifier 74. The feedback circuit 75 is configured to set different DC and AC gain.

The underlying principle used by the measuring circuit 7 is based on a parasitic capacitance 88 formed between isolated ground of the output socket 14 and ground of the internal circuit 2 ("secondary ground"). Protective earth PE and secondary ground are considered as being connected via a low impedance 89 of approx. 1 Milliohm (1 mΩ), as symbolized by element 89. Thereby the current loop is closed at least with respect to AC current.

Moreover, via this high impedance 89 the secondary ground of the parasitic capacitance is connected to protective earth at the AE electrode 83. This means: Whenever the +12/-12 Volt toggle voltage 87 toggles, current flows through the parasitic capacitance 88 and capacitor C5 of the capacitive voltage divider 71, which is translated to a proportional voltage by capacitor C7 of the capacitive voltage divider 71. This voltage is amplified by the operational amplifier 74 which preferably is of the type having a very low bias current, in particular a JFET type. The feedback circuit 75 provides for a DC gain of unity and a rather high AC gain of 100 at the relevant frequency as defined by the toggle voltage 87 which is about 250 Hz in the depicted embodiment. Thereby ample amplification of the useful signal is provided. Further, noise and ripple introduced by the switching frequency of the internal circuit 2 is removed by the blocking filter 76. To this end, a capacitor C10 is provided which interacts with the operational amplifier 74 in a manner to act as a bandpass filter for blocking high frequencies such as a switching frequency of a power supply (not shown) for the internal circuitry 2.

The resulting filtered output signal from the output of the operational amplifier 74 is supplied via line 77 to an input of an analog/digital converter (ADC) 78, whose digital output signal is in turn supplied to a digital threshold detector 79. Its threshold is selected such that an output signal is generated if the electrosurgical instrument with its cable 15 is connected to the active electrode of the socket 14, and no signal is generated if the measured capacitance is below the threshold since no instrument with its cable is connected to the active electrode. The resulting signal is indicative of the status whether an electrosurgical instrument 16 with its cable 15 is connected or not, and it is fed by line 70 to the control unit 10, thereby providing an automatic detection of the presence of the electrosurgical instrument 16.

## Claims

1. An electrosurgical generator designed to output a high-frequency alternating voltage to an electrosurgical instrument (16), comprising a control unit (10) and an inverter (3) for high voltage, the inverter being configured to generate a high-frequency alternating voltage which is fed via an output connection to an output socket (14) for connection of the electrosurgical instrument (16), wherein a galvanic separator (33) is provided isolating the output socket from the control unit, and wherein a detection unit is provided being configured to detect when the electrosurgical instrument is plugged in the output socket,
wherein the detection unit is a capacitive detector (4) configured for detecting a capacitance of a plugged-in cable (15) of the electrosurgical instrument (16).

2. The electrosurgical generator as claimed in claim 1, wherein a connection of the capacitive detector (4) to the output connection (13) is configured to be made at a conductor for a, preferably active, electrode (AE) of the output connection (13).

3. The electrosurgical generator as claimed in claim 1 or 2, wherein the capacitive detector (4) is configured for detecting a binary state, namely a first high capacitance state indicating the cable (15) being plugged-in and a second low capacitance state indicating no cable being plugged-in .

4. The electrosurgical generator as claimed in any of the preceding claims, wherein the capacitive detector (4) comprises a capacitive pick-up (5) arranged at the output socket (14) interacting with a measuring circuit (7) being configured to check a measured capacitance against a reference value.

5. The electrosurgical generator as claimed in claim 4, wherein the measuring circuit (7) is galvanically isolated from the capacitive pick-up (5), preferably by a separate protective series capacitor (6).

6. The electrosurgical generator as claimed in the preceding claim, wherein the protective series capacitor (6) is configured as a printed circuit board structure (60), said printed circuit board structure (60) preferably comprising two plates (61, 62) being opposite from each other and being isolated from each other.

7. The electrosurgical generator as claimed in claim 4, wherein the measuring circuit (7) is connected to the output socket (14), and an output signal emitted by the measuring circuit (7) is fed to the control unit (10) via a protected connection (19).

8. The electrosurgical generator as claimed in any of the claims 4 to 6, wherein the measuring circuit (7) is configured for direct capacitance measurement, wherein the measuring circuit (7) is preferably formed by an integrated circuit.

9. The electrosurgical generator as claimed in any of the claims 4 to 6, wherein the measuring circuit (7) is configured for indirect capacitance measurement.

10. The electrosurgical generator as claimed in the preceding claim, wherein the measuring circuit (7) is configured for determining a capacitive leakage current at the output socket (14).

11. The electrosurgical generator as claimed in claim 9, wherein a low voltage AC signal is injected into the output connection (13), and the measuring circuit (7) is configured for measuring said low voltage AC signal.

12. The electrosurgical generator as claimed in the preceding claim, wherein the measuring circuit (7) comprises a capacitive voltage divider (71) connected between the output connection (13) and a local ground at the output socket (14), said capacitive voltage divider (71) generating a low voltage signal to be measured.

13. The electrosurgical generator as claimed in the preceding claim, wherein further a resistive voltage divider (73) is provided for providing a bias voltage for the measuring circuit (7).

14. The electrosurgical generator as claimed in any of claims 11 to 13, wherein the measuring circuit (7) comprises an operational amplifier (74) being configured for setting different DC gain and AC gain, wherein the AC gain is at least ten times, preferably more than fifty times, higher than the DC gain which is preferably set to substantially unity.

15. The electrosurgical generator as claimed in any of the preceding claims, wherein the measurement circuit (7) comprises a blocking filter (76), preferably a notch filter, configured for blocking a switching frequency of a power supply of the electrosurgical generator.

## Patentansprüche

1. Elektrochirurgischer Generator, der ausgelegt ist, eine Hochfrequenzwechselspannung zu einem elektrochirurgischen Instrument (16) auszugeben, und eine Steuereinheit (10) und einen Wechselrichter (3) für Hochspannung umfasst, wobei der Wechselrichter konfiguriert ist, eine Hochfrequenzwechselspannung zu erzeugen, die mittels einer Ausgangsverbindung in eine Ausgangsbuchse (14) zur Verbindung des elektrochirurgischen Instruments (16) eingespeist wird, wobei ein galvanischer Separator (33) vorgesehen ist, der die Ausgangsbuchse von der Steuereinheit isoliert, und eine Detektionseinheit vorgesehen ist, die konfiguriert ist, zu detektieren, wann das elektrochirurgische Instrument in die Ausgangsbuchse eingesteckt ist,
wobei die Detektionseinheit ein kapazitiver Detektor (4) ist, der zum Detektieren einer Kapazität eines eingesteckten Kabels (15) des elektrochirurgischen Instruments (16) konfiguriert ist.

2. Elektrochirurgischer Generator nach Anspruch 1, wobei eine Verbindung des kapazitiven Detektors (4) mit der Ausgangsverbindung (13) konfiguriert ist, bei einem Leiter für eine bevorzugt aktive Elektrode (AE) der Ausgangsverbindung (13) hergestellt zu werden.

3. Elektrochirurgischer Generator nach Anspruch 1 oder 2, wobei der kapazitive Detektor (4) zum Detektieren eines Binärzustands, nämlich eines ersten Hochkapazitätszustands, der angibt, dass das Kabel (15) eingesteckt ist, und eines zweiten Niederkapazitätszustands, der angibt, dass kein Kabel eingesteckt ist, konfiguriert ist.

4. Elektrochirurgischer Generator nach einem der vorhergehenden Ansprüche, wobei der kapazitive Detektor (4) eine kapazitive Aufnahme (5) umfasst, die bei der Ausgangsbuchse (14) angeordnet ist und mit einer Messschaltung (7) interagiert, die konfiguriert ist, eine gemessene Kapazität gegen einen Bezugswert zu prüfen.

5. Elektrochirurgischer Generator nach Anspruch 4, wobei die Messschaltung (7) von der kapazitiven Aufnahme (5) galvanisch isoliert ist, bevorzugt durch einen getrennten in Reihe geschalteten Schutzkondensator (6).

6. Elektrochirurgischer Generator nach dem vorhergehenden Anspruch, wobei der in Reihe geschaltete Schutzkondensator (6) als eine Struktur (60) einer gedruckten Leiterplatte konfiguriert ist und die Struktur (60) einer gedruckten Leiterplatte bevorzugt zwei Platten (61, 62) umfasst, die einander gegenüberliegen und voneinander isoliert sind.

7. Elektrochirurgischer Generator nach Anspruch 4, wobei die Messschaltung (7) mit der Ausgangsbuchse (14) verbunden ist und ein Ausgangssignal, das durch die Messschaltung (7) abgegeben wird, mittels einer geschützten Verbindung (19) in die Steuereinheit (10) eingespeist wird.

8. Elektrochirurgischer Generator nach einem der Ansprüche 4 bis 6,
wobei die Messschaltung (7) zur direkten Kapazitätsmessung konfiguriert ist und die Messschaltung (7) bevorzugt durch eine integrierte Schaltung gebildet ist.

9. Elektrochirurgischer Generator nach einem der Ansprüche 4 bis 6, wobei die Messschaltung (7) zur indirekten Kapazitätsmessung konfiguriert ist.

10. Elektrochirurgischer Generator nach dem vorhergehenden Anspruch, wobei die Messschaltung (7) zum Bestimmen eines kapazitiven Leckstroms bei der Ausgangsbuchse (14) konfiguriert ist.

11. Elektrochirurgischer Generator nach Anspruch 9, wobei ein Niederspannungswechselstromsignal in die Ausgangsverbindung (13) eingespeist wird und die Messschaltung (7) zum Messen des Niederspannungswechselstromsignals konfiguriert ist.

12. Elektrochirurgischer Generator nach dem vorhergehenden Anspruch, wobei die Messschaltung (7) einen kapazitiven Spannungsteiler (71) umfasst, der zwischen der Ausgangsverbindung (13) und einer lokalen Masse bei der Ausgangsbuchse (14) verbunden ist, und der kapazitive Spannungsteiler (71) ein Niederspannungssignal erzeugt, das gemessen werden soll.

13. Elektrochirurgischer Generator nach dem vorhergehenden Anspruch, wobei ferner ein ohmscher Spannungsteiler (73) zum Bereitstellen einer Vorspannung für die Messschaltung (7) bereitgestellt ist.

14. Elektrochirurgischer Generator nach einem der Ansprüche 11 bis 13, wobei die Messschaltung (7) einen Operationsverstärker (74) umfasst, der zum Einstellen verschiedener Gleichstrom- und Wechselstromverstärkungen konfiguriert ist, und die Wechselstromverstärkung mindestens zehnmal, bevorzugt mehr als fünfzigmal höher als die Gleichstromverstärkung ist, die bevorzugt auf im Wesentlichen eins gesetzt ist.

15. Elektrochirurgischer Generator nach einem der vorhergehenden Ansprüche, wobei die Messschaltung (7) ein Sperrfilter (76), bevorzugt ein Kerbfilter umfasst, das zum Blockieren einer Schaltfrequenz einer Stromversorgung des elektrochirurgischen Generators konfiguriert ist.

## Revendications

1. Générateur électrochirurgical conçu pour délivrer une tension alternative haute fréquence à un instrument électrochirurgical (16), comprenant une unité de commande (10) et un onduleur (3) pour haute tension, l'onduleur étant configuré pour générer une tension alternative haute fréquence qui est appliquée, via une connexion de sortie, à une prise de sortie (14) permettant de connecter l'instrument électrochirurgical (16), un séparateur galvanique (33) étant agencé pour isoler la prise de sortie de l'unité de commande, et une unité de détection étant agencée et configurée pour détecter lorsque l'instrument électrochirurgical est branché sur la prise de sortie,
l'unité de détection consistant en un détecteur capacitif (4) configuré pour détecter une capacité d'un câble (15) branché de l'instrument électrochirurgical (16).

2. Générateur électrochirurgical selon la revendication 1, une connexion du détecteur capacitif (4) à la connexion de sortie (13) étant configurée pour être réalisée au niveau d'un conducteur pour une électrode, de préférence active, (AE) de la connexion de sortie (13) .

3. Générateur électrochirurgical selon la revendication 1 ou 2, le détecteur capacitif (4) étant configuré pour détecter un état binaire, à savoir un premier état de capacité élevée indiquant que le câble (15) est branché et un deuxième état de faible capacité indiquant qu'aucun câble n'est branché.

4. Générateur électrochirurgical selon l'une quelconque des revendications précédentes, le détecteur capacitif (4) comprenant un capteur capacitif (5) placé au niveau de la prise de sortie (14) qui interagit avec un circuit de mesure (7) qui est configuré pour contrôler une capacité mesurée en la comparant à une valeur de référence.

5. Générateur électrochirurgical selon la revendication 4, le circuit de mesure (7) étant isolé galvaniquement du capteur capacitif (5), de préférence par un condensateur de protection (6) distinct monté en série.

6. Générateur électrochirurgical selon la revendication précédente, le condensateur de protection (6) monté en série étant configuré sous forme d'une structure de carte de circuit imprimé (60) de préférence comprenant deux plaques (61, 62) en regard l'une de l'autre et isolées l'une de l'autre.

7. Générateur électrochirurgical selon la revendication 4, le circuit de mesure (7) étant connecté à la prise de sortie (14), et un signal de sortie émis par le circuit de mesure (7) étant appliqué à l'unité de commande (10) via une connexion protégée (19).

8. Générateur électrochirurgical selon l'une quelconque des revendications 4 à 6, le circuit de mesure (7) étant configuré pour une mesure directe de la capacité, le circuit de mesure (7) étant de préférence formé d'un circuit intégré.

9. Générateur électrochirurgical selon l'une quelconque des revendications 4 à 6, le circuit de mesure (7) étant configuré pour une mesure indirecte de la capacité.

10. Générateur électrochirurgical selon la revendication précédente, le circuit de mesure (7) étant configuré pour déterminer un courant de fuite capacitif au niveau de la prise de sortie (14).

11. Générateur électrochirurgical selon la revendication 9, un signal alternatif basse tension étant injecté dans la connexion de sortie (13), et le circuit de mesure (7) étant configuré pour mesurer ledit signal alternatif basse tension.

12. Générateur électrochirurgical selon la revendication précédente, le circuit de mesure (7) comprenant un diviseur de tension capacitif (71) connecté entre la connexion de sortie (13) et une terre locale au niveau de la prise de sortie (14), ledit diviseur de tension capacitif (71) générant un signal basse tension à mesurer.

13. Générateur électrochirurgical selon la revendication précédente, un diviseur de tension résistif (73) étant en outre agencé pour fournir une tension de polarisation pour le circuit de mesure (7).

14. Générateur électrochirurgical selon l'une quelconque des revendications 11 à 13, le circuit de mesure (7) comprenant un amplificateur opérationnel (74) qui est configuré pour régler un gain en courant continu et un gain en courant alternatif différents, le gain en courant alternatif étant au moins dix fois, de préférence plus de cinquante fois, supérieur au gain en courant continu qui est de préférence réglé sur un gain sensiblement unité.

15. Générateur électrochirurgical selon l'une quelconque des revendications précédentes, le circuit de mesure (7) comprenant un filtre de blocage (76), de préférence un filtre coupe-bande, configuré pour bloquer une fréquence de découpage d'une alimentation électrique du générateur électrochirurgical.
